# EUROPÄISCHE PATENTSCHRIFT

(11) **EP 3 307 709 B1**
(45) Veröffentlichungstag und Bekanntmachung des Hinweises auf die Patenterteilung: **13.02.2019**
(21) Anmeldenummer: 16728958.6
(22) Anmeldetag: 14.06.2016
(51) Int. Cl.: C07C 267/00, C08G 18/02

(54) **VERFAHREN ZUR HERSTELLUNG VON POLYMEREN CARBODIIMIDEN UNTER ZUSATZ VON CÄSIUMSALZEN, POLYMERE CARBODIIMIDE UND DEREN VERWENDUNG**
METHOD FOR THE PREPARATION OF POLYMER CARBODIIMIDES WITH THE ADDITION OF CAESIUM SALTS, POLYMERIC CARBODIIMIDES AND THEIR USE
PROCEDE DE FABRICATION DE CARBODIIMIDES POLYMERES A L'AIDE DE SELS DE CESIUM, CARBODIIMIDES POLYMERES ET LEUR UTILISATION

(30) Priorität: 15.06.2015 EP 15172169
(43) Veröffentlichungstag der Anmeldung: 18.04.2018
(73) Patentinhaber: LANXESS Deutschland GmbH, 50569 Köln (DE)
(72) Erfinder: LAUFER, Wilhelm, 67158 Ellerstadt (DE); HERD, Oliver, 42369 Wuppertal (DE); SPERBER, Rolf, 42369 Wuppertal (DE)
(86) Internationale Anmeldenummer: PCT/EP2016/063601
(87) Internationale Veröffentlichungsnummer: WO 2016/202781

(56) Entgegenhaltungen:
- EP-A1- 0 628 541
- EP-A1- 2 803 660
- WO-A1-2005/111136

## Beschreibung

Die vorliegende Erfindung betrifft Verfahren zur Herstellung von polymeren Carbodiimiden unter Zusatz von Cäsiumsalzen, die über dieses Verfahren hergestellten polymeren Carbodiimide sowie deren Verwendung als Hydrolyseschutzmittel in Polyurethan(PU)-basierten Systemen, vorzugsweise thermoplastisches TPU, PU-Klebstoffen, PU-Gießharzen, PU-Elastomeren oder PU-Schäumen

Carbodiimide haben sich in vielen Anwendungen bewährt, z.B. als Hydrolyseschutzmittel für thermoplastische Kunststoffe, Ester-basierte Polyole, Polyurethane, Triglyceride und Schmierstofföle etc.

Die Synthese der Carbodiimide erfolgt nach dem Stand der Technik ausgehend von Isocyanaten, die durch basische oder heterocyclische Katalyse unter CO₂-Abspaltung carbodiimidsiert werden. Dabei können mono- oder polyfunktionelle Isocyanate zu monomeren oder polymeren Carbodiimiden umgesetzt werden.

Die üblicherweise verwendeten Katalysatoren sind Alkali- oder Erdalkaliverbindungen, wie z.B. Alkalialkoholate sowie heterocyclische Verbindungen, die Phosphor enthalten, wie z.B. in der Zeitschrift Angewandte Chemie beschrieben, siehe Angew. Chem. 1962, 74, 801-806 und Angew. Chem. 1981, 93, 855-866.

Die Herstellung von sterisch gehinderten polymeren Carbodiimiden nach dem Stand der Technik gelingt nur mit Hilfe von Phosphor-haltigen (z. B. Phospholene) Katalysatoren, siehe EP-A-2803660, WO 2005/111136 oder EP-A 0628541. Die vollständige Entfernung dieser Phosphor-haltigen Katalysatoren ist technisch nicht möglich. Da Carbodiimide vorzugsweise bei der Herstellung von Polyurethanen eingesetzt werden, ist die Anwesenheit von Phosphor, selbst im Spurenbereich, extrem störend und ist daher zu vermeiden.

Aufgabe der vorliegenden Erfindung war es daher, ein verbessertes Verfahren bereitzustellen, welches die Herstellung von polymeren Carbodiimiden in hohen Ausbeuten ermöglicht und über dies zudem polymere Carbodiimide, die frei von organischen Phosphorverbindungen sind, so dass diese bei der Herstellung und/oder Stabilisierung von PU-Systemen eingesetzt werden kann.

Überraschenderweise wurde nun gefunden, dass die vorgenannten Aufgaben erfüllt sind, wenn polymere Carbodiimide durch die Umsetzung von Isocyanat-Gruppen-enthaltenden Verbindungen in Gegenwart von mindestens einem basischen Cäsiumsalz bei Temperaturen zwischen 120 bis 220° C, vorzugsweise 160 bis 200°C, ganz besonders bevorzugt 180 bis 200°C umgesetzt (carbodiimidisiert) werden.

Gegenstand der vorliegenden Erfindung ist daher ein Verfahren zur Herstellung von polymeren Carbodiimiden der Formel (I)

R¹-R²-(-N=C=N-R²-)ₘ-R¹ (I),

in der
m einer ganzen Zahl von 2 bis 500, bevorzugt 3 bis 20, ganz besonders bevorzugt 4 bis 10 entspricht,
R² = C₁ - C₁₈-Alkylen, C₅-C₁₈-Cycloalkylen, Arylen, C₇-C₁₈⁻ Alkylarylen und/oder C₇-C₁₈-Aralkylen, vorzugsweise Alkylarylen und/oder C₇-C₁₈- Aralkylen
   und
R¹ = -NCO, -NCNR², -NHCONHR⁴, -NHCONR⁴R⁵ oder -NHCOOR⁶ ist,
wobei in R¹ unabhängig voneinander R⁴ und R⁵ gleich oder verschieden sind und einen C₁C₆-Alkyl-, C₆-C₁₀-Cycloalkyl- oder C₇-C₁₈-Aralkylrest darstellen und R⁶ einen Polyester- oder einen Polyamidrest oder -(CH₂)ₕ-O-[(CH₂)ₖ-O]_{g}-R⁷ bedeutet,
mit h = 1-3, k= 1-3, g = 0-12 und
R⁷= H oder C₁-C₄-Alkyl,
wonach Isocyanat-Gruppen-enthaltende Verbindungen der Formel (II)

   O=C=N-R²-R¹ (II)
gegebenenfalls in Anwesenheit von Isocyanat-Gruppen-enthaltende Verbindungen der Formel (III)

   O=C=N-R² (III)
wobei R² und R¹ die oben genannte Bedeutung haben,
in Gegenwart von mindestens einem basischen Cäsiumsalz bei Temperaturen zwischen 120 bis 220° C , vorzugsweise 160 bis 200°C, ganz besonders bevorzugt 180 bis 200°C umgesetzt (carbodiimidisiert) werden.

Als basische Cäsiumsalzen im Sinne der Erfindung werden vorzugsweise Cäsiumcarbonat und/oder Cäsiumalkoholate, vorzugsweise Cäsiummethylat, eingesetzt.

Die basischen Cäsiumssalze werden dabei vorzugsweise in einer Konzentration von 0,1 bis 20 Gew.-%, besonders bevorzugt 1 bis 5 Gew.-%, ganz besonders bevorzugt 2 bis 4 Gew.-% eingesetzt.

Besonders bevorzugte Isocyanat-Gruppen-enthaltende Verbindungen der Formel (II) sind die nachstehend genannten Verbindungen, die einzeln oder in der nachstehend genannten Kombination eingesetzt werden:
(IIa), (IIb), (IIc) oder (IId) alleine oder (IIa) und (IIb) gemeinsam,
wobei diese Verbindungen folgenden Formeln entsprechen

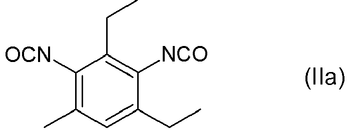

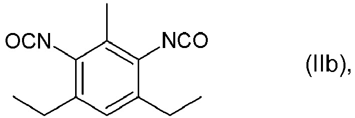

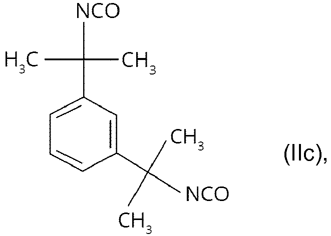

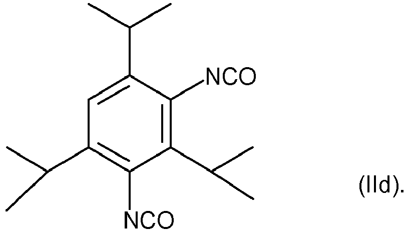

Bei den Verbindungen der Formel (III) handelt es sich vorzugsweise um Di- und /oder
Triisopropylphenylisocyanat sowie Isopropenyldimethylbenzylisocyanat.

In einer bevorzugten Ausführungsform des erfindungsgemäßen Verfahrens werden die basischen Cäsiumsalze im Anschluss an die Carbodiimidisierung abfiltiriert und / oder durch Extraktion mittels eines Lösemittels, bevorzugt Wasser und/oder Alkohol, abgetrennt.

Die Carbodiimidisierung kann sowohl in Substanz als auch in einem Lösemittel durchgeführt werden. Als Lösemittel werden vorzugsweise C₇ - C₂₂- Alkylbenzole, Paraffinöle, Polyethylenglykoldimethylether, Ketone oder Lactone eingesetzt.

Wenn die Reaktionsmischung den gewünschten Gehalt an NCO-Gruppen, entsprechend einem mittleren Kondensationsgrad von m = 2 bis 500, bevorzugt 3 bis 20, ganz besonders bevorzugt 4 bis 10, besitzt, wird die Polycarbodiimidisierung vorzugsweise gestoppt.

In einer Ausführungsform der vorliegenden Erfindung wird hierzu die Temperatur der Reaktionsmischung auf 50 - 120 °C, bevorzugt 60 - 100 °C, besonders bevorzugt auf 80 - 90 °C reduziert und die basischen Cäsiumsalze werden mittels Filtration oder Extraktion abgetrennt. In einer bevorzugten Herstellungsvariante der erfindungsgemäßen Carbodiimide werden anschließend die überschüssigen Isocyanat-Gruppen-enthaltenden Verbindungen bei Temperaturen von 150 - 200° C, bevorzugt 160 - 180°C abdestilliert.

In einer weiteren Ausführungsform der vorliegenden Erfindung werden anschließend die freien endständigen Isocyanatgruppen der Carbodiimide mit aliphatischen und/oder aromatischen Aminen, Alkoholen und/oder Alkoxypolyoxyalkylenalkoholen, vorzugsweise in einem geringem Überschuss an -NH-, -NH₂- und/oder -OH-Gruppen, gegebenenfalls in Anwesenheit eines dem Fachmann bekannten Katalysators, vorzugsweise tert. Aminen oder Organozinn-Verbindungen, besonders bevorzugt DBTL (Dibutylzinndilaurat oder DOTL (Dioctylzinndilaurat), abreagiert. Das Stoffmengen-Verhältnis von Aminen, Alkoholen und/oder Alkoxypolyoxyalkylenalkoholen zu polymeren Carbodiimiden nach Formel (I) liegt vorzugsweise bei 1,005 - 1,05 : 1, besonders bevorzugt bei 1,01 - 1,03 : 1, bezogen auf die vorhandenen N=C=O-Gruppen.

In einer weiteren Ausführungsform der vorliegenden Erfindung wird zur Unterbrechung der Carbodiimidisierung die Temperatur der Reaktionsmischung auf 50 - 120°C, bevorzugt 60 - 100 °C, besonders bevorzugt auf 80 - 90 °C reduziert und gegebenenfalls nach Zugabe eines Lösemittels, bevorzugt ausgewählt aus der Gruppe der C₇ - C₂₂-Alkylbenzole, besonders bevorzugt Toluol, werden die freien endständigen Isocyanatgruppen der Carbodiimide mit aliphatischen und/oder aromatischen Aminen, Alkoholen und/oder Alkoxypolyoxyalkylenalkoholen vorzugsweise in einem geringem Überschuss an -NH-, -NH₂- und/oder -OH-Gruppen, gegebenenfalls in Anwesenheit eines dem Fachmann bekannten PU-Katalysators, vorzugsweise tert. Aminen oder Organozinn-Verbindungen, besonders bevorzugt DBTL (Dibutylzinn dilaurat oder DOTL (Dioctylzinndilaurat), abreagiert. Das Stoffmengen-Verhältnis von Aminen, Alkoholen und/oder Alkoxypolyoxyalkylenalkoholen zu den polymeren Carbodiimiden gemäß Formel (I) liegt vorzugsweise bei 1,005 - 1,05 : 1, besonders bevorzugt bei 1,01 - 1,03 : 1, bezogen auf die vorhandenen N=C=O-Gruppen.

In einer weiteren Ausführungsform der Erfindung erfolgt die Herstellung der erfindungsgemäßen polymeren Carbodiimide nach Formel (I) durch eine partielle, vorzugsweise <50%-ige, bevorzugt < 40 % Endfunktionalisierung der freien NCO-Gruppen in den Isocyanat-Gruppen-enthaltende Verbindungen der Formel (I) mit R¹ = - NCO mit primären oder sekundären Aminen oder Alkoholen und/oder Alkoxypolyoxyalkylenalkoholen in den Diisocyanaten und anschließende Carbodiimidisierung unter Abspaltung von Kohlendioxid bei Temperaturen von 80°C bis 200 °C in Gegenwart von Cäsium-Salze und gegebenenfalls Lösungsmittel.

Vorzugsweise werden die erfindungsgemäßen Carbodiimide nach ihrer Herstellung gereinigt. Die Reinigung der Rohprodukte kann destillativ und/oder mittels Extraktion mit Lösemitteln erfolgen. Als geeignete Lösemittel für die Aufreinigung können vorzugsweise, C₇ - C₂₂-Alkylbenzole, Paraffinöle, Alkohole, Ketone oder Ester eingesetzt werden. Dabei handelt es sich um handelsübliche Lösemittel.

Ein weiterer Gegenstand der vorliegenden Erfindung sind polymere Carbodiimide nach Formel (I) erhältlich nach dem erfindungsgemäßen Verfahren.

Ein weiterer Gegenstand der vorliegenden Erfindung sind Stabilisatoren, enthaltend mindestens 90% an polymeren Carbodiimiden nach Formel (I), vorzugsweise erhältlich nach dem erfindungsgemäßen Verfahren, mit einem Anteil von weniger als 1 ppm an organischen Phosphorverbindungen, wie vorzugsweise Phospholenoxide.

In einer weiteren bevorzugten Ausführungsform der Erfindung weisen die erfindungsmäßen Stabilisatoren vorzugsweise maximal 1000 ppm, bevorzugt maximal 100 ppm und besonders bevorzugt maximal 10 ppm an Cäsiumsalze aufweist.

Die Stabilisatoren ermöglichen vor allem einen hervorragenden Hydrolyseschutz.

Ein weiterer Gegenstand der vorliegenden Erfindung sind zudem Verfahren zur Herstellung von Polyurethanen (PU), bevorzugt thermoplastischen Polyurethanen, wonach die Umsetzung der Polyole, vorzugsweise der Polyesterpolyole, mit den Isocyanaten in Gegenwart des erfindungsgemäßen polymeren Carbodiimides durchgeführt wird und/oder die erfindungsgemäßen polymeren Carbodiimide im Anschluss an die Umsetzung zum Polyurethan zugesetzt werden.

In einer weiteren bevorzugten Ausführungsform der Erfindung wird das Verfahren in Anwesenheit von PU-Katalysatoren und Hilf- und oder Zusatzstoffen durchgeführt.

Die Herstellung der Polyurethane erfolgt dabei vorzugsweise, wie in WO 2005/111136 A1 beschrieben.

Polyurethane entstehen durch Polyadditionsreaktion von Polyisocyanaten mit mehrwertigen Alkoholen, den Polyolen, vorzugsweise Polyesterpolyolen, nahezu quantitativ. Die Verknüpfung erfolgt durch die Reaktion einer Isocyanatgruppe (-N=C=O) eines Moleküls mit einer Hydroxygruppe (-OH) eines anderen Moleküls unter Bildung einer Urethangruppe (-NH-CO-O-).

Der Reaktionsverlauf zwischen Diisocyanat und Polyol ist abhängig von dem Molverhältnis der Komponenten. Zwischenstufen mit gewünschtem Durchschnittsmolekulargewicht und gewünschten Endgruppen können durchaus erhalten werden. Diese Zwischenstufen können dann zu einem späteren Zeitpunkt mit einem Diol oder Diamin umgesetzt (kettenverlängert) werden, wobei dann das gewünschte Polyurethan bzw. Polyurethan-Polyharnstoff-Hybrid gebildet wird. Die Zwischenstufen werden im Allgemeinen als Prepolymer bezeichnet.

Geeignete Polyole für die Herstellung von Prepolymeren sind Polyalkylenglykolether, Polyetherester oder Polyester mit endständigen Hydroxylgruppen (Polyesterpolyole).

Bei den Polyolen im Sinne der Erfindung handelt es sich um Verbindungen, die vorzugsweise ein Molekulargewicht (in g/mol) von bis zu 2000, bevorzugt im Bereich von 500 bis 2000 und besonders bevorzugt im Bereich von 500 bis 1000 aufweisen.

Der Begriff Polyol im Sinne der Erfindung umfasst dabei sowohl Diole als auch Triole, wie auch Verbindungen mit mehr als drei Hydroxylgruppen je Molekül. Die Verwendung von Triolen ist besonders bevorzugt.

Bevorzugte Polyole sind Polyesterpolyole und/oder Polyetheresterpolyole.

Vorteilhaft ist es, wenn das Polyol eine OH-Zahl von bis zu 200, vorzugsweise zwischen 20 und 150 und besonders bevorzugt zwischen 50 und 115, aufweist.

Insbesondere eignen sich Polyesterpolyole, die Reaktionsprodukte von verschiedenen Polyolen mit aromatischen oder aliphatischen Dicarbonsäuren und/oder Polymeren von Lactonen sind.

Bevorzugt hierbei sind aromatische Dicarbonsäuren, welche zur Bildung geeigneter Polyesterpolyole verwendet werden können. Besonders bevorzugt sind hier Terephthalsäure, Isophthalsäure, Phthalsäure, Phtalsäureanhydrid sowie substituierte Dicarbonsäureverbindungen mit Benzolkern.

Als aliphatische Dicarbonsäuren sind solche bevorzugt, welche zur Bildung geeigneter Poylesterpolyole verwendet werden können, besonders bevorzugt Sebacinsäure, Adipinsäure und Glutarsäure.

Als Polymere von Lactonen sind solche bevorzugt, welche zur Bildung geeigneter Poylesterpolyole verwendet werden können, besonders bevorzugt Polycaprolacton.

Sowohl bei den Dicarbonsäuren als auch bei den Polymeren von Lactonen handelt es sich um handelsübliche Substanzen.

Besonders bevorzugt sind auch solche Polyole, welche zur Bildung geeigneter Polyesterpolyole verwendet werden können, ganz besonders bevorzugt Ethylenglykol, Butandiol, Neopentylglykol, Hexandiol, Propylenglykol, Dipropylenglykol, Diethylenglykol und Cyclohexandimethanol.

In einer weiteren bevorzugten Ausführungsform der Erfindung handelt es sich bei den Polyolen um Polyetheresterpolyole.

Hierfür sind die Reaktionsprodukte von verschiedenen vorhergehend genannten Polyolen mit aromatischen oder aliphatischen Dicarbonsäuren und/oder Polymeren von Lactonen (z.B. Polycaprolacton) bevorzugt.

Bei den im Sinne der Erfindungen eingesetzten Polyolen handelt es sich um handelsübliche Verbindungen, die bei der Firma Bayer MaterialScience AG unter dem Handelsnamen Baycoll® oder Desmophen® erhältlich sind.

Als Diisocyanate sind aromatische und aliphatische Diisocyanate bevorzugt. Besonders bevorzugt sind Toluol-2,4-diisocyanat, Toluol-2,6-diisocyanat, Phenylendiisocyanat, 4,4-Diphenylmethandiisocyanat, Methylen-bis(4-phenylisocynat), Naphtalen-1,5-diisocyanat, Tetramethylen-1,4.diisocyanat und/oder Hexamethylen-1,6-diisocyanat, ganz besonders bevorzugt Toluol-2,4-diisocyanat und Toluol-2,6-diisocyanat.

Bei den im Sinne der Erfindungen eingesetzten Diisocyanaten handelt es sich um handelsübliche Verbindungen, die z.B. bei der Firma Bayer MaterialScience AG unter dem Handelsnamen Desmodur® erhältlich sind.

In einer weiteren Ausführungsform der Erfindung enthält die Zusammensetzung zusätzlich mindestens ein Diamin und/oder Diol.

Als Diamine, welche für die Kettenverlängerung eingesetzt werden, sind 2-Methylpropyl-3,5-diamino-4-chlorobenzoat, Bis-(4,4'-amino-3-chlorophenyl)-methan, 3,5-Dimethylthio-2,4-toluylendiamin, 3,5-Dimethylthio-2,4-toluylendiamin, 3,5-Diethyl-2,4-toluylendiamin, 3,5-Diethyl-2,6-toluylendiamin, 4,4'-Methylen-bis-(3-chloro-2,6-diethylanilin) und 1,3-Propandiol-bis(4-aminobenzoat) bevorzugt.

Als Diole sind Butandiol, Neopentylglykol, Hexandiol, Propylenglykol, Dipropylenglykol, Diethylenglykol und/oder Cyclohexandimethanol bevorzugt.

Bei den im Sinne der Erfindung zur Kettenverlängerung eingesetzten Diaminen oder Diolen handelt es sich um handelsübliche Verbindungen, die bei der Firma Rheinchemie Rheinau GmbH unter dem Handelsnamen Addolink® erhältlich sind.

Als Katalysatoren werden vorzugsweise Dibutylzinndilaurate oder Triethylendiamin in Dipropylenglycol eingesetzt.

Bei den im Sinne der Erfindungen eingesetzten Katalysatoren handelt es sich um handelsübliche Verbindungen, die bei der Firma Rheinchemie Rheinau GmbH unter dem Handelsnamen Addocat® erhältlich sind.

In einer besonders bevorzugten Ausführungsform der vorliegenden Erfindung wird das erfindungsgemäße polymere Carbodiimid nach Formel (I) in einer Menge von 0,1 bis 2 Gew.-%, bevorzugt 0,5 bis 1,5 Gew.-%, besonders bevorzugt 1,0 bis 1,5 Gew.-% bezogen auf die Gesamtmischung, eingesetzt.

Ein weiterer Gegenstand der vorliegenden Erfindung ist zudem die Verwendung des erfindungsgemäßen polymere Carbodiimide nach Formel (I) in Verfahren zur Herstellung von Polyurethanen als Hydrolysestabilisator.

Die nach diesem Verfahren hergestellten Polyurethan(PU)-basierten Systeme zeichnen sich durch eine exzellente Hydrolysestabilität aus.

Ein weiterer Gegenstand der vorliegenden Erfindung ist zudem die Verwendung der erfindungsgemäßen polymeren Carbodiimide gemäß Formel (I) zum Hydrolyseschutz, vorzugsweise in Polyurethanen.

Der Rahmen der Erfindung erfasst alle oben stehenden und im Folgenden aufgeführten allgemeinen oder in Vorzugsbereichen genannten Restedefinitionen, Indizes, Parameter und Erläuterungen untereinander, also auch zwischen den jeweiligen Bereichen und Vorzugsbereichen in beliebiger Kombination.

Die nachfolgenden Beispiele dienen der Erläuterung der Erfindung, ohne dabei limitierend zu wirken.

### Ausführungsbeispiele:

- **Beispiel 1:**: Herstellung eines polymeren Carbodiimids durch Umsetzung der Verbindung der Formel (IIc) mit Cäsiumcarbonat (erfindungsgemäß).
- **Beispiel 2:**: Herstellung eines polymeren Carbodiimids durch Umsetzung der Verbindung der Formel (IIc) mit Kalium-Methanolat (Vergleich).
- **Beispiel 3:**: Herstellung eines polymeren Carbodiimids durch Umsetzung der Verbindung der Formel (IIc) mit Kaliumcarbonat (Vergleich).
- **Beispiel 4:**: Herstellung eines polymeren Carbodiimids durch Umsetzung der Verbindung der Formel (IIc) mit Natriumcarbonat (Vergleich).
- **Beispiel 5:**: Herstellung eines polymeren Carbodiimids durch Umsetzung der Verbindung der Formel (IIc) mit Phospholenoxid (Vergleich).

### Allgemeine Herstellvorschrift für die Beispiele 1 - 5:

Es wurden 30 g der Isocyanat-Gruppen-enthaltenden Verbindung der Formel (IIc) in einen 100 ml-Dreihalskolben eingewogen, der mit Innenthermometer, Rückflusskühler und Schutzgaseinlass ausgestattet war, und daraufhin wurden für die Beispiele 1 bis 4 0.9 g (3 Gew.%) und für Beispiel 5 0,03 g (0,1 Gew.%) des jeweiligen Katalysators gemäß Tabelle 1 zugefügt. In der Aufheizphase gab man einen leichten Argonstrom über die Dampfphase. Bei beginnender CO₂-Entwicklung wurde das Schutzgas abgestellt. Man ließ 3 h bei 190 °C (Beispiele 1 und 5) bzw. 12 h bei 190 °C (Beispiel 2) und 6 h bei 190°C (Beispiele 3 und 4) kräftig rühren und filtrierte anschließend das auf ca. 100°C abgekühlte Reaktionsgemisch.

**Tabelle 1: Ausbeuten der Synthese des Carbodiimids**

| **Beispiel** | **Katalysator** | **T [°C]** | **Dauer [h]** | **Carbodiimid** | **Isocyanat** | **Nebenprodukte** |
|---|---|---|---|---|---|---|
| 1 | Cäsiumcarbonat | 190 | 3 | > 98% | <1,0% | < 1,0% |
| 2 | K-Methanolat | 190 | 12 | < 60 % | > 30 % | > 5,0% |
| 3 | Kaliumcarbonat | 190 | 6 | < 1 % | 99 % | n. b. |
| 4 | Natriumcarbonat | 190 | 6 | 0 % | 100 % | - |
| 5 | Phospolenoxid | 190 | 3 | > 95% | < 1,0% | > 1,0% |

Überraschenderweise zeigt das Cäsiumcarbonat für die Carbodiimidisierung eine hohe Katalysatoraktivität und führte bereits nach 3 Stunden Reaktionszeit zu Ausbeuten von über 98 % und ist damit deutlich besser als die Synthese über K-Methanolat oder K- bzw. Na-Carbonat.

Zudem kann der erfindungsgemäße Katalysator einfach durch Filtration abgetrennt werden, während im Fall einer Katalyse mittels eines Phospor-haltigen Katalysators (Methyl-Phospholenoxid) eine aufwendige Destillation im Vakuum zur Abtrennung erfolgen muss.

### Herstellung von esterbasierten PU-Schmelzklebstoffen (Hotmelts) und dessen Charakterisierung

### Beispiel 6:

Es wurde ein Schmelzklebstoff auf Basis von linearem Copolyester mit primären Hydroxylfunktionen und einem mittleren molekularen Gewicht von 3500g/mol (Dynacoll® 7360) hergestellt und dieser entsprechend der nachfolgenden Tabelle additiviert:
Als Carbodiimide wurde eingesetzt:
(A) 2 Gew. % polymeres Carbodiimid der Formel (I) mit m = 4 - 5 und R¹ = -NHCOOR⁶
   wobei R⁶ ein Polyethylenglykolrest ist, hergestellt durch die Katalyse mit Cäsiumcarbonat (erfindungsgemäß, in Anlehnung an Beispiel 1 jedoch mit Polyethylenglykol endfunktionalisiert),
   und R² = 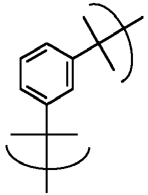
   Charakterisierung: Keine organische Phosphorverbindung nachweisbar (< 1 ppm Phosphor)
(B) 2 Gew. % polymeres Carbodiimid der Formel (I) mit m = 4 - 5 und R¹ = -NHCOOR⁶
   wobei R⁶ ein Polyethylenglykolrest ist, hergestellt durch die Katalyse mit Methylphospholenoxid (Vergleich, siehe auch Verfahren aus WO-A 2005/111136),
   und R² = 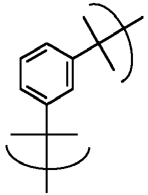
   Charakterisierung: Reste an Phosphor nachweisbar.

Alle Mengenangaben sind in Gew.-%, bezogen auf die Gesamtmischung.

Der Schmelzklebstoff wurde wie folgt hergestellt:
Zunächst wurde der lineare Copolyester mit primären Hydroxylfunktionen für 30 Minuten - bei 120 °C evakuiert. Anschließend erfolgte die Zugabe von 11,67 Gew.% Diphenylmethandiisocyanat (MDI), bezogen auf die Gesamtformulierung, und es wurde für 60 Minuten bei 120 °C zum Polyurethanklebstoff umgesetzt.

Anschließend wurden die jeweiligen, in der Tabelle 2 angegebenen Carbodiimide in den Schmelzklebstoff eingearbeitet und eine Einwirkzeit der Additive von 1 Stunde sichergestellt.

Die so hergestellten und additivierten Schmelzklebstoffe (Hot-Melt) wurden in einer Kartusche einer Temperaturalterung bei 130°C für 48 Stunden unterworfen. Die Temperaturalterung wurde in einer Alukartusche (Licht- und Feuchtigkeitsdicht) abgefüllt und im Umluftofen für 48 Stunden bei 130 °C gealtert.

Nach der Alterung wurde die der Proben visuell beurteilt.

Die Ergebnisse der Messungen sind in der Tabelle 2 zusammengestellt:

**Tabelle 2:**

| **Carbodiimid** | **Charakterisierung** |
|---|---|
| Beispiel 6A (erf.) | Keine Schaumbildung, Keine oder sehr geringe Blasenbildung, |
| Beispiel 6B (V) | Schaum- bzw. starke Blasenbildung, |

| | |
|---|---|
| V = Vergleichsbeispiel; erf. = erfindungsgemäß | |

### Resümee:

Diese Versuche zeigen, dass durch die Verwendung des erfindungsgemäßen phosphorfreien Carbodiimides keine nennenswerte störende Nebeneffekte bezüglich Schäumen auftreten. Im Gegenteil dazu zeigen Carbodiimide, die mit Phospholenoxid katalysiert wurden und noch Spuren von Phosphor-organische Verbindungen beinhalten die entsprechend angegebenen Nachteile der Schaumbildung.

## Patentansprüche

1. Verfahren zur Herstellung von polymeren Carbodiimiden der Formel (I)
R¹-R²-(-N=C=N-R²-)ₘ-R¹ (I),
in der
m einer ganzen Zahl von 2 bis 500, bevorzugt 3 bis 20, ganz besonders bevorzugt 4 bis 10 entspricht,
R² = C₁ - C₁₈-Alkylen, C₅-C₁₈-Cycloalkylen, Arylen, C₇-C₁₈⁻Alkylarylen und/oder C₇-C₁₈- Aralkylen, vorzugsweise Alkylarylen und/oder C₇-C₁₈- Aralkylen
und
R¹ = -NCO, -NCNR², -NHCONHR⁴, -NHCONR⁴R⁵ oder -NHCOOR⁶ ist,
wobei in R¹ unabhängig voneinander R⁴ und R⁵ gleich oder verschieden sind und einen C₁C₆-Alkyl-, C₆-C₁₀-Cycloalkyl- oder C₇-C₁₈-Aralkylrest darstellen und R⁶ einen Polyester- oder einen Polyamidrest oder -(CH₂)ₕ-O-[(CH₂)ₖ-O]_{g}-R⁷ bedeutet,
mit h = 1-3, k= 1-3, g = 0-12 und
R⁷= H oder C₁-C₄-Alkyl, **dadurch gekennzeichnet, dass** Isocyanat-Gruppen-enthaltende Verbindungen der Formel (II)
O=C=N-R²-R¹ (II),
gegebenenfalls in Anwesenheit von Isocyanat-Gruppen-enthaltende Verbindungen der Formel (III)
O=C=N-R² (III)
wobei R¹ und R² die oben genannte Bedeutung haben,
in Gegenwart von mindestens einem basischen Cäsiumsalz bei Temperaturen zwischen 120 bis 220° C, vorzugsweise 160 bis 200°C, ganz besonders bevorzugt 180 bis 200°C umgesetzt (carbodiimidisiert) werden.

2. Verfahren nach Anspruch 1, **dadurch gekennzeichnet, dass** als basisches Cäsiumsalz Cäsiumcarbonat und/oder Cäsiumalkoholat, vorzugsweise Cäsiummethylat, eingesetzt wird.

3. Verfahren nach Anspruch 1 oder 2, **dadurch gekennzeichnet, dass** als Isocyanat-Gruppen-enthaltende Verbindungen die folgenden Verbindungen eingesetzt werden:
(IIa), (IIb), (IIc) oder (IId) alleine oder (IIa) und (IIb) gemeinsam, wobei diese Verbindungen folgenden Formeln entsprechen

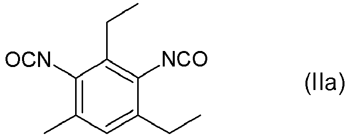

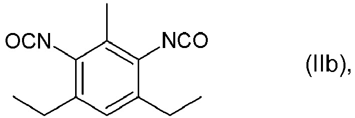

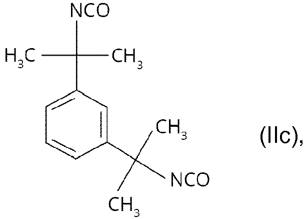

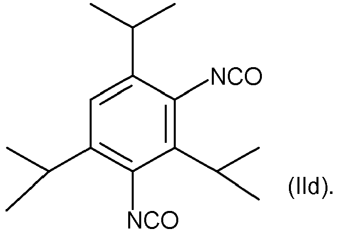

4. Verfahren nach einem der Ansprüche 1 bis 3, **dadurch gekennzeichnet, dass** die basischen Cäsiumsalze in einer Konzentration von 0,1 bis 20 Gew.%, bevorzugt 1 bis 5 Gew.-%, besonders bevorzugt 2 bis 4 Gew.-%, bezogen auf die Gesamtmischung eingesetzt werden.

5. Verfahren nach einem der Ansprüche 1 bis 4, **dadurch gekennzeichnet, dass** die basischen Cäsiumsalze im Anschluss an die Carbodiimidisierung abfiltiriert und / oder mittels eines Lösemittel, bevorzugt Wasser und/oder Alkohol, durch Extraktion abgetrennt werden.

6. Verfahren nach einem der Ansprüche 1 bis 5, **dadurch gekennzeichnet, dass** die Carbodiimisierung in einem Lösemittel stattfindet.

7. Verfahren nach Anspruch 6, **dadurch gekennzeichnet, dass** als Lösemittel C₇ - C₂₂.-Alkylbenzole eingesetzt werden.

8. Stabilisator, enthaltend mindestens 90% an polymeren Carbodiimiden hergestellt nach einem der Ansprüche 1 bis 7 und weniger als 1 ppm an organischen Phosphorverbindungen.

9. Verfahren zur Herstellung von Polyurethanen, bevorzugt thermoplastischen Polyurethanen, **dadurch gekennzeichnet, dass** man die Umsetzung der Polyesterpolyole mit den Isocyanaten in Gegenwart von polymeren Carbodiimiden hergestellt nach einem der Ansprüche 1 bis 7 durchführt oder die polymeren Carbodiimide hergestellt nach einem der Ansprüche 1 bis 7 im Anschluss an die Umsetzung zugesetzt.

10. Verfahren nach Anspruch 9, **dadurch gekennzeichnet, dass** das polymere Carbodiimid in einer Menge von 0,1 bis 2 Gew.-%, bevorzugt 0,5 bis 1,5 Gew.-%, besonders bevorzugt 1,0 bis 1,5 Gew.-%, bezogen auf die Gesamtmischung, eingesetzt wird.

11. Verwendung der polymeren Carbodiimide hergestellt nach einem der Ansprüche 1 bis 7 in einem Verfahren zur Herstellung von Polyurethanen.

12. Verwendung der polymeren Carbodiimide hergestellt nach einem der Ansprüche 1 bis 7 zum Hydrolyseschutz, vorzugsweise in Polyurethanen.

## Claims

1. Process for producing polymeric carbodiimides of formula (I)
R¹-R²-(-N=C=N-R²-)ₘ-R¹ (I),
in which
m represents an integer from 2 to 500, preferably 3 to 20, very particularly preferably 4 to 10,
R² = C₁-C₁₈-alkylene, C₅-C₁₈-cycloalkylene, arylene, C₇-C₁₈-alkylarylene and/or C₇-C₁₈-aralkylene, preferably alkylarylene and/or C₇-C₁₈-aralkylene
and
R¹ = -NCO, -NCNR², -NHCONHR⁴, -NHCONR⁴R⁵ or -NHCOOR⁶,
wherein in R¹ independently of one another R⁴ and R⁵ are identical or different and represent a C₁-C₆-alkyl, C₆-C₁₀-cycloalkyl or C₇-C₁₈-aralkyl radical and R⁶ represents a polyester or a polyamide radical or -(CH₂)ₕ-O-[(CH₂)ₖ-O]_{g}-R⁷,
where h = 1-3, k = 1-3, g = 0-12 and
R⁷= H or C₁-C₄-alkyl, **characterized in that** isocyanate-containing compounds of formula (II)
O=C=N-R²-R¹ (II),
optionally in the presence of isocyanate-containing compounds of formula (III)
O=C=N-R² (III)'
wherein R¹ and R² are as defined above,
are converted (carbodiimidized) in the presence of at least one basic cesium salt at temperatures between 120°C to 220°C, preferably 160°C to 200°C, very particularly preferably 180°C to 200°C.

2. Process according to Claim 1, **characterized in that** the basic cesium salt employed is cesium carbonate and/or cesium alkoxide, preferably cesium methoxide.

3. Process according to Claim 1 or 2, **characterized in that** the isocyanate-containing compounds employed are the following compounds:
(IIa), (IIb), (IIc) or (IId) alone or (IIa) and (IIb) together, wherein these compounds correspond to the following formulae

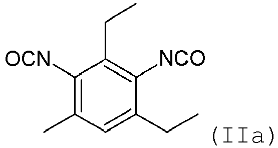

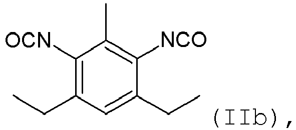

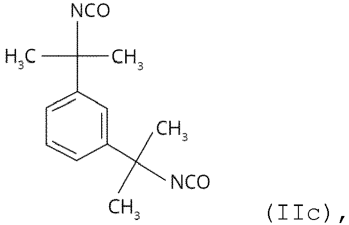

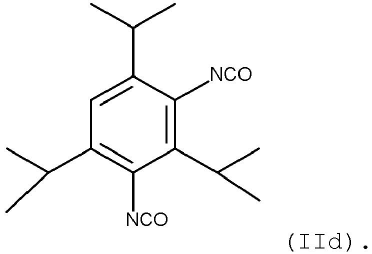

4. Process according to any of Claims 1 to 3, **characterized in that** the basic cesium salt are employed in a concentration of 0.1 to 20 wt%, preferably 1 to 5 wt%, particularly preferably 2 to 4 wt%, based on the overall mixture.

5. Process according to any of Claims 1 to 4, **characterized in that** following the carbodiimidization the basic cesium salts are filtered off and/or removed by extraction using a solvent, preferably water and/or alcohol.

6. Process according to any of Claims 1 to 5, **characterized in that** the carbodiimidization takes place in a solvent.

7. Process according to Claim 6, **characterized in that** the solvents employed are C₇-C₂₂-alkylbenzenes.

8. Stabilizer containing at least 90% of polymeric carbodiimides produced according to any of Claims 1 to 7 and less than 1 ppm of organic phosphorus compounds.

9. Process for producing polyurethanes, preferably thermoplastic polyurethanes, **characterized in that** the reaction of the polyester polyols with the isocyanates is performed in the presence of polymeric carbodiimides produced according to any of Claims 1 to 7 or the polymeric carbodiimides produced according to any of Claims 1 to 7 are added following the reaction.

10. Process according to Claim 9, **characterized in that** the polymeric carbodiimide is employed in an amount of 0.1 to 2 wt%, preferably 0.5 to 1.5 wt%, particularly preferably 1.0 to 1.5 wt%, based on the overall mixture.

11. Use of the polymeric carbodiimides produced according to any of Claims 1 to 7 in a process for producing polyurethanes.

12. Use of the polymeric carbodiimides produced according to any of Claims 1 to 7 for hydrolysis protection, preferably in polyurethanes.

## Revendications

1. Procédé de fabrication de carbodiimides polymères de formule (I)
R¹-R²-(-N=C=N-R²-)ₘ-R¹ (I),
dans laquelle
m correspond à un nombre entier de 2 à 500, de préférence de 3 à 20, de manière tout particulièrement préférée de 4 à 10,
R² = alkylène en C₁-C₁₈, cycloalkylène en C₅-C₁₈, arylène, alkylarylène en C₇-C₁₈ et/ou aralkylène en C₇-C₁₈, de préférence alkylarylène et/ou aralkylène en C₇-C₁₈,
et
R¹ = -NCO, -NCNR², -NHCONHR⁴, -NHCONR⁴R⁵ ou -NHCOOR⁶, dans R¹, R⁴ et R⁵ étant indépendamment l'un de l'autre identiques ou différents et représentant un radical alkyle en C₁-C₆, cycloalkyle en C₆-C₁₀ ou aralkyle en C₇-C₁₈, et R⁶ signifiant un radical polyester ou polyamide ou -(CH₂)ₕ-O-[(CH₂)ₖ-O]_{g}-R⁷,
avec h = 1 à 3, k = 1 à 3, g = 0 à 12, et
R⁷ = H ou alkyle en C₁-C₄, **caractérisé en ce que** des composés contenant des groupes isocyanate de formule (II)
O=C=N-R²-R¹ (II)
sont mis en réaction (carbodiimidés) éventuellement en présence de composés contenant des groupes isocyanate de formule (III)
O=C=N-R² (III)
dans laquelle R¹ et R² ont la signification indiquée précédemment,
en présence d'au moins un sel de césium basique à des températures comprises entre 120 et 220 °C, de préférence de 160 à 200 °C, de manière tout particulièrement préférée de 180 à 200 °C.

2. Procédé selon la revendication 1, **caractérisé en ce que** du carbonate de césium et/ou un alcoolate de césium, de préférence le méthylate de césium, sont utilisés en tant que sel de césium basique.

3. Procédé selon la revendication 1 ou 2, **caractérisé en ce que** les composés suivants sont utilisés en tant que composés contenant des groupes isocyanate :
(IIa), (IIb), (IIc) ou (IId) seuls ou (IIa) et (IIb) ensemble, ces composés correspondant aux formules suivantes :

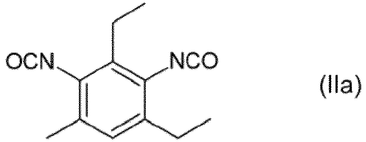

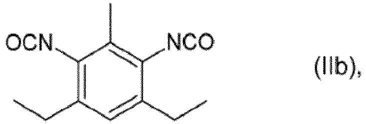

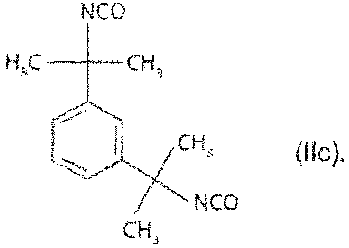

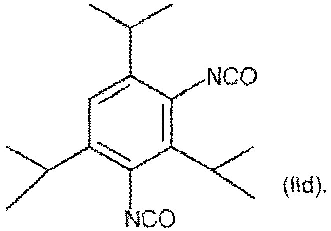

4. Procédé selon l'une quelconque des revendications 1 à 3, **caractérisé en ce que** les sels de césium basiques sont utilisés en une concentration de 0,1 à 20 % en poids, de préférence de 1 à 5 % en poids, de manière particulièrement préférée de 2 à 4 % en poids, par rapport au mélange total.

5. Procédé selon l'une quelconque des revendications 1 à 4, **caractérisé en ce que** les sels de calcium basiques sont filtrés après la carbodiimidation et/ou séparés par extraction au moyen d'un solvant, de préférence de l'eau et/ou un alcool.

6. Procédé selon l'une quelconque des revendications 1 à 5, **caractérisé en ce que** la carbodiimidation a lieu dans un solvant.

7. Procédé selon la revendication 6, **caractérisé en ce que** des alkylbenzènes en C₇-C₂₂ sont utilisés en tant que solvant.

8. Stabilisateur, contenant au moins 90 % de carbodiimides polymères fabriqués selon l'une quelconque des revendications 1 à 7 et moins de 1 ppm de composés de phosphore organiques.

9. Procédé de fabrication de polyuréthanes, de préférence de polyuréthanes thermoplastiques, **caractérisé en ce que** la réaction des polyester-polyols avec les isocyanates est réalisée en présence de carbodiimides polymères fabriqués selon l'une quelconque des revendications 1 à 7, ou les carbodiimides polymères fabriqués selon l'une quelconque des revendications 1 à 7 sont ajoutés après la réaction.

10. Procédé selon la revendication 9, **caractérisé en ce que** le carbodiimide polymère est utilisé en une quantité de 0,1 à 2 % en poids, de préférence de 0,5 à 1,5 % en poids, de manière particulièrement préférée de 1,0 à 1,5 % en poids, par rapport au mélange total.

11. Utilisation des carbodiimides polymères fabriqués selon l'une quelconque des revendications 1 à 7 dans un procédé de fabrication de polyuréthanes.

12. Utilisation des carbodiimides polymères fabriqués selon l'une quelconque des revendications 1 à 7 pour la protection contre l'hydrolyse, de préférence dans des polyuréthanes.
